# EUROPEAN PATENT SPECIFICATION

(11) **EP 2 709 659 B1**
(45) Date of publication and mention of the grant of the patent: **29.08.2018**
(21) Application number: 12722712.2
(22) Date of filing: 16.05.2012
(51) Int. Cl.: G01N 33/569, A61K 39/00, C07K 16/14

(54) **METHOD FOR DETECTING INFECTIONS**
VERFAHREN ZUM NACHWEIS VON INFEKTIONEN
PROCÉDÉ DE DÉTECTION D'INFECTIONS

(30) Priority: 16.05.2011 IE 20110235
(43) Date of publication of application: 26.03.2014
(73) Proprietor: National University of Ireland, Maynooth, Maynooth, County Kildare (IE)
(72) Inventor: DOYLE, John Martin, Maynooth County Kildare (IE); WALSHE, Kieran, Maynooth County Kildare (IE); GORDON, Natasha, Maynooth County Kildare (IE); KAVANAGH, Kevin, Maynooth County Kildare (IE); GALLAGHER, Lorna, Maynooth County Kildare (IE)
(74) Representative: D Young & Co LLP
(86) International application number: PCT/EP2012/059133
(87) International publication number: WO 2012/156452

(56) References cited:
- WO-A2-2005/093036
- US-A1- 2006 079 580
- HOWARD DH ET AL: "Hydroxamate Siderophores of Histoplasma capsulatum", INFECTION AND IMMUNITY, vol. 68, no. 4, April 2000 (2000-04), pages 2338-2343, XP008153546,
- BUYER J S ET AL: "MONOCLONAL ANTIBODIES TO FERRIC PSEUDOBACTIN, THE SIDEROPHORE OF PLANT GROWTH-PROMOTING PSEUDOMONAS PUTIDA B10", APPLIED AND ENVIRONMENTAL MICROBIOLOGY, AMERICAN SOCIETY FOR MICROBIOLOGY, US, vol. 56, no. 2, 1 February 1990 (1990-02-01), pages 419-424, XP000943280, ISSN: 0099-2240
- FOX M; GRAY G; KAVANAGH K; LEWIS C; DOYLE S.: "Detection of Aspergillus fumigatus mycotoxins: immunogen synthesis and immunoassay development", J MICROBIOL METHODS, vol. 56, no. 2, 2004, pages 221-230, XP008153539, cited in the application
- JEGOROV ALEXANDR ET AL: "Nonribosomal cyclic peptides: specific markers of fungal infections", JOURNAL OF MASS SPECTROMETRY, WILEY, CHICHESTER, GB, vol. 41, no. 5, 1 May 2006 (2006-05-01), pages 563-576, XP002561629, ISSN: 1076-5174, DOI: 10.1002/JMS.1042 [retrieved on 2006-06-13] cited in the application
- M. PETRIK ET AL: "68Ga-Siderophores for PET Imaging of Invasive Pulmonary Aspergillosis: Proof of Principle", THE JOURNAL OF NUCLEAR MEDICINE, vol. 51, no. 4, 1 April 2010 (2010-04-01), pages 639-645, XP055032734, ISSN: 0161-5505, DOI: 10.2967/jnumed.109.072462 cited in the application
- C. M. MARAGOS ET AL: "Development of monoclonal antibodies for the fusarin mycotoxins", FOOD ADDITIVES AND CONTAMINANTS., vol. 25, no. 1, 29 November 2007 (2007-11-29), pages 105-114, XP55295200, GB ISSN: 0265-203X, DOI: 10.1080/02652030701518098

## Description

The present invention relates to a method for detecting infections caused by or associated with siderophore-secreting microorganisms.

*Aspergillus fumigatus* is an opportunistic pathogen and causes severe disease and mortality in immunocompromised individuals (Dagenais, T. R. and Keller, N. P. (2009) Pathogenesis of Aspergillus fumigatus in Invasive Aspergillosis. Clin Microbiol Rev 22(3): 447-465). An infection by an *Aspergillus spp.* is called aspergillosis and this type of infection can be divided into degrees of severity, where invasive aspergillosis (IA), sometimes referred to as invasive pulmonary aspergillosis (IPA), is the most fatal type of infection (Latge, J. P. (1999) Aspergillus fumigatus and aspergillosis. Clin Microbiol Rev 12(2): 310-350). The mortality rate associated with IA ranges from 60 - 90 % depending on the primary condition causing the immunosuppression (Latge, J. P. (1999) Aspergillus fumigatus and aspergillosis. Clin Microbiol Rev 12(2): 310-350). Other infections include chronic cavity pulmonary aspergillosis (CCPA), and allergic bronchopulmonary aspergillosis (ABPA) and saprophytic aspergilloma (Buckingham, S. J. and Hansell, D. M. (2003) Aspergillus in the lung: diverse and coincident forms. Eur Radiol 13(8): 1786-1800). Also, antifungal drugs can be toxic at high levels, are expensive, and must be administered as early as possible to ensure successful patient response. Therefore, the sensitive and specific diagnosis of IA is essential to initiate treatment as soon as possible.

Detection of fungal disease in humans is difficult and failure to diagnose quickly can lead to morbidity and death, particularly those with IA caused by *A. fumigatus.* For example, results from blood culture are positive in less than 5% of cases of IA (Jegorov, A., Hajduch, M., Sulc, M. and Havlicek, V. (2006) Nonribosomal cyclic peptides: specific markers of fungal infections. J Mass Spectrom 41(5): 563-576). Diagnosis is often erroneous because several other filamentous fungi have a similar appearance under the microscope. Distinguishing *Aspergillus spp.* from *Fusarium spp.* or *Scedosporium spp.* is important in determining antifungal susceptibility, identifying drug-resistant species, selecting appropriate antifungal therapy, and maintaining surveillance and epidemiological tracking of IA. Current non-invasive methods for detecting *A. fumigatus* infection are by X-ray or CT scan of the lungs, whereby a mycelial mass that has become so well-established is detectable (Maschmeyer G, Haas A, Cornely OA. (2007) Invasive aspergillosis: epidemiology, diagnosis and management in immunocompromised patients. Drugs. 67(11):1567-1601). One additional complication with treatment is that many patients are initially treated for bacterial infection, with detection, or suspicion, of fungal infection occurring only after initial treatment has failed. At this stage an underlying fungal infection may have resulted in a poor patient outcome.

Non-invasive tests for the diagnosis of IA include (i) galactomannan detection, (ii) (1-3)β-glucan detection and (iii) PCR amplification of specific regions of the fungal genome.
(i) Galactomannan is a heat-stable heteropolysaccharide present in the cell wall of most *Aspergillus* and *Penicillium* species (Verdaguer, V., Walsh, T. J., Hope, W. and Cortez, K. J. (2007) Galactomannan antigen detection in the diagnosis of invasive aspergillosis. Expert Rev Mol Diagn 7(1): 21-32). Using the ELISA sandwich method, detection is confirmed due to the multiple immunoreactive epitopes on galactomannan. The sensitivity of the assay can be compromised due to the fact that the antibody binding requires four or more galactofuranoside epitopes for a positive result and, therefore, a sub-optimal amount of binding may not be enough for valid detection (Hope, W. W., Walsh, T. J. and Denning, D. W. (2005) Laboratory diagnosis of invasive aspergillosis. Lancet Infect Dis 5(10): 609-22). In addition, in humans, galactomannan forms immune complexes that are quickly removed from the circulation, further limiting the sensitivity of these assays (Yeo SF, Wong B. (2002) Current status of nonculture methods for diagnosis of invasive fungal infections. Clin Microbiol Rev. 15(3):465-484). More importantly, the sensitivity of the galactomannan ELISA is reduced in patients receiving anti-fungal drugs as prophylaxis or empiric treatment on the day of testing (Marr KA, Laverdiere M, Gugel A, Leisenring W. (2005) Antifungal therapy decreases sensitivity of the Aspergillus galactomannan enzyme immunoassay. Clin Infect Dis. 40(12):1762-1769). The galactomannan detection assay is commercially available as Platelia™ Aspergillus (Bio-Rad Laboratories, Marnes-La-Coquette, France and Bio-Rad Laboratories, Hercules, CA, USA).
(ii) (1-3)β-glucan is present in the cell wall of most fungi (Maertens, J., Theunissen, K., Lodewyck, T., Lagrou, K. and Van Eldere, J. (2007) Advances in the serological diagnosis of invasive Aspergillus infections in patients with haematological disorders. Mycoses 50 Suppl 1: 2-17), hindering the use of this molecule as an *A. fumigatus-specific* analyte. As the (1-3)β-glucan assay is carried out using serum specimens, consideration needs to be given to the presence of serine protease inhibitors found in human plasma, which need to be inactivated before use in the assay (Hope, W. W., Walsh, T. J. and Denning, D. W. (2005) Laboratory diagnosis of invasive aspergillosis. Lancet Infect Dis 5(10): 609-622). The β-glucan assay is available in three commercial forms: Fungitell™ (Associates of Cape Cod Inc., East Falmout, MA, USA), Fungi-Tec G™ (Seikagaku Kogyo Corporation, Tokyo, Japan) and Wako-WB003 (Wako Pure Chemical Industries, Osaka, Japan).
(iii) PCR detection of *A. fumigatus* can be carried out by the use of pan-fungal primers that are used to amplify conserved regions in the DNA of fungal species. Ribosomal DNA (rDNA) is the most common target, specifically the 18S, 28S and 5.8S genes (White, P. L., Linton, C. J., Perry, M. D., Johnson, E. M. and Barnes, R. A. (2006) The evolution and evaluation of a whole blood polymerase chain reaction assay for the detection of invasive aspergillosis in hematology patients in a routine clinical setting. Clin Infect Dis 42(4): 479-486). The prevalence of *A. fumigatus* DNA in the environment (e.g., blood collection tubes) necessitates the provision of specialised collection tubes, by *A. fumigatus* PCR assay manufacturers, to ensure assay specificity (http://www.mvconostica.co.uk/mvcxtra). Thus, PCR detection of IA is far from satisfactory for multiple reasons.

Therefore, conventional diagnosis of IA is either (a) laborious (culturing of infectious agent), (b) subject to poor sensitivity or specificity of detection (galactomannan or β-glucan detection) or (c) unable to distinguish between live/pathogenic versus dead organisms (fungal PCR).

It has been suggested that detection by mass spectrometry of low molecular mass compounds or metabolites (e.g., siderophores), which may be secreted by actively growing infectious microorganisms and which should not be present in the uninfected host, may represent an alternative method to diagnose infectious, particularly fungal, disease (Jegerov et al., 2006). However, this has yet to be demonstrated, proven to be of use, or used, in a clinical situation. Moreover, mass spectrometric detection of non-ribosomal peptides (e.g. siderophores) is complex, very expensive and requires expensive instrumentation and expert operator involvement. In addition, it would be expected that specimen extraction and optimisation, to remove interfering compounds, would be required for the application of this technique for disease diagnosis. Furthermore, the rapid uptake of siderophores by fungi, *in vitro* and *in vivo* - allied to the presence of siderophore-binding, defensive proteins (e.g., siderocalins and lipocalins) in animals (Fluckinger M, Haas H, Merschak P, Glasgow BJ, Redl B. (2004) Human tear lipocalin exhibits antimicrobial activity by scavenging microbial siderophores. Antimicrob Agents Chemother. 48(9):3367-3372) - would lead one skilled in the art to conclude that siderophore detection would not be a valid strategy for diagnosis of IA or IPA because those analytes would not be readily available for detection.

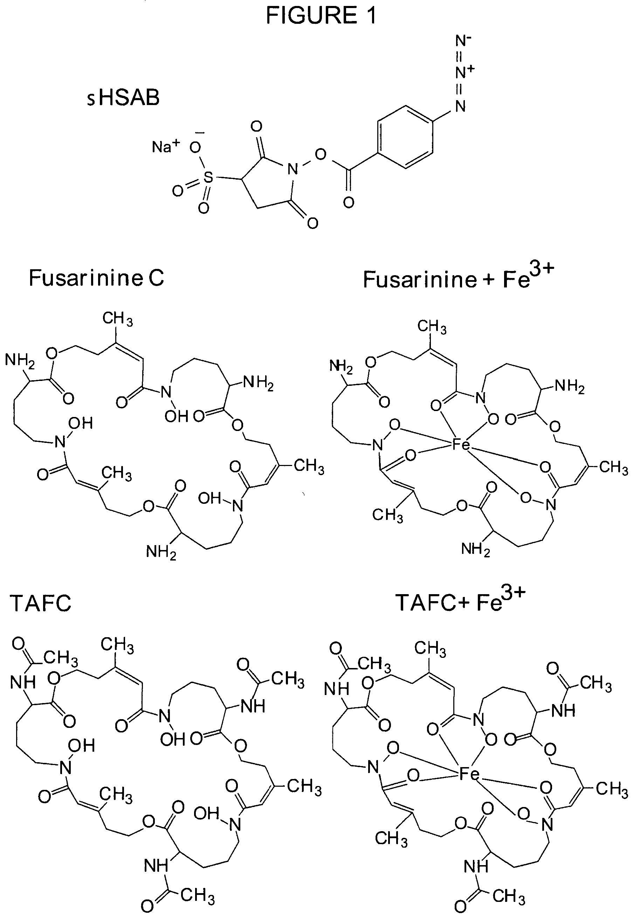

Siderophores (e.g., triacetylfusarinine C (TAFC), fusarinine C (FusC) or ferricrocin (FC); Figure 1) are iron-chelating peptides produced by fungi and bacteria to scavenge free iron (Fe³⁺) in the immediate environment and facilitate its transport into the cell (Haas H. (2003) Molecular genetics of fungal siderophore biosynthesis and uptake: the role of siderophores in iron uptake and storage. Appl Microbiol Biotechnol. 62(4):316-330). The *in vitro* production of siderophores by *A. fumigatus,* grown in the presence of diluted human serum was demonstrated - using a colorimetric reagent (CAS) to detect the siderophores in these specimens, which were further diluted prior to CAS assay (Hissen AH, Chow JM, Pinto LJ, Moore MM. (2004) Survival of Aspergillus fumigatus in serum involves removal of iron from transferrin: the role of siderophores. Infect Immun. 72:1402-1408). Hissen *et al.* (2004) did not discuss use of the CAS assay for detection of siderophores as indicators of infection. Furthermore, no demonstration of the diagnostic potential of *A. fumigatus* siderophore detection in human, or other animal, biological fluids was present in these publications.

Siderophore biosynthesis is essential for the virulence of *A. fumigatus* in animal model systems, for example the murine model of IPA (Schrettl, M., Bignell, E., Kragl, C., Joechl, C., Rogers, T., Arst, H. N., Jr., Haynes, K. and Haas, H. (2004) Siderophore biosynthesis but not reductive iron assimilation is essential for Aspergillus fumigatus virulence. J Exp Med 200(9): 1213-9). Siderophore (TAFC and fusarinine C; Figure 1) biosynthesis is activated under iron-limiting conditions to scavenge for extracellular Fe³⁺. Ferricrocin (FC) is primarily involved in iron storage within *A. fumigatus.* In *A. fumigatus,* FusC is converted to TAFC by enzyme-mediated acetylation via the action of the enzyme SidG. It has been proposed (Petrik M, Haas H, Dobrozemsky G, Lass-Flörl C, Helbok A, Blatzer M, Dietrich H, Decristoforo C. (2010) 68Ga-Siderophores for PET imaging of invasive pulmonary aspergillosis: proof of principle. J. Nuclear Medicine 51(4) 639-645) that radiolabelled TAFC and to a lesser extent, FC, have an application in the *in vivo* detection of IPA. These authors radiolabelled purified TAFC and FC and determined via PET if either ⁶⁸Ga-labelled siderophore was taken up by *A. fumigatus* during rodent infection. Upon administration, it was found that ⁶⁸Ga-TAFC was rapidly secreted into the urine of uninfected mice, while ⁶⁸Ga-FC was retained in serum. This teaches that TAFC, and also fusarinine C, would not be good serum biomarkers of IPA as they would be likely to be rapidly secreted by animals. In a rat model of IPA, ⁶⁸Ga-TAFC was taken up to the greatest extent by fungal mycelia in severely infected rats, with rapid uptake into infected lungs accompanied by rapid renal excretion.

US Patent No. 2006/0079580 is directed to the production and characterization of a mutant strain of Aspergillus fumigatus. In the absence of any working embodiments, this document speculates that TAFC could theoretically or potentially be detectable as a biomarker, by a method involving noncompetitive (i.e., sandwich) ELISA detection of the analyte. However, no experiments were performed to support this contention as stated in US Patent No. 2006/0079580. Moreover, no experimental data is presented in US Patent No. 2006/0079580. We put forward that the proposed method of US Patent No. 2006/0079580 would not allow the detection of small molecules such as TAFC due to epitope restriction.

In summary, as outlined above, conventional diagnoses of infections caused by or associated with siderophore-secreting microorganisms have significant disadvantages.

It is an object of the present invention to mitigate or eliminate the disadvantages associated with conventional methods of diagnosing infections caused by or associated with siderophore-secreting microorganisms.

It is also an object of the present invention to provide a method for detecting infections caused by or associated with FusC-secreting microorganisms, including, but not limited to, infections caused by or associated with *Aspergillus fumigatus,* which is simple, accurate and cost-efficient.

It is also an object of the present invention to provide a method for detecting fusarinine C, referred to hereinafter as FusC, which is simple, accurate and cost-efficient.

FusC-secreting fungi may be selected from the group comprising *Rhizopogon luteolus Inv, Rhizopogon luteolus Yum, Suillus luteus, Suillus granulatus, Scleroderma verrucosum, Phanerochaete chryosporium, Trametes versicolor, Pleurotus ostreatus, Penicillium chrysogenum, Cryptococcus neoformans, Fusarium roseum, Histoplasma capsulatum* and from the genus *Aspergillus* including the strains *Aspergillus flavus, Aspergillus terreus, Aspergillus nidulans* and *Aspergillus fumigatus.* The detection of FusC secreted by any one of *Cryptococcus neoformans, Aspergillus nidulans* and *Aspergillus fumigatus* is preferred, most preferably siderophores secreted by *Aspergillus fumigatus.* Accordingly, the method of the invention is particularly useful for detecting an infection caused by or associated with *Aspergillus fumigatus.*

*Aspergillus fumigatus* infections include, but are not limited to, forms of aspergillosis, optionally selected from but not limited to, invasive aspergillosis (IA), often referred to as invasive pulmonary aspergillosis (IPA), chronic cavity pulmonary aspergillosis (CCPA), allergic bronchopulmonary aspergillosis (ABPA) and saprophytic aspergilloma.

According to a first general aspect of the invention, there is provided a method for detecting fusarinine C (FusC) and/or detecting infections caused by or associated with FusC-secreting microorganisms, preferably an Aspergillus fumigatus infection, more preferably invasive aspergillosis (IA), in a biological sample of a subject, the method comprising:
providing a solid support having either bound FusC or a conjugate thereof, or bound anti-FusC antibody;
reacting the bound FusC or a conjugate thereof, with an anti-FusC antibody, and a biological sample of a subject; or reacting the bound anti-FusC antibody with a FusC or conjugate thereof, and a biological sample of a subject;
detecting and/or quantifying the presence of the FusC in the biological sample,
wherein the presence of FusC in the biological sample is indicative of infection caused by or associated with FusC-secreting microorganisms, preferably *Aspergillus fumigatus* infection, more preferably invasive aspergillus (IA), and wherein the anti-FusC antibody was raised against an immunogen comprising FusC covalently coupled to a carrier protein.

Ideally, this method is a competitive immunossay optionally selected from the group comprising enzyme-linked immunosorbent assay (ELISA), immunochromatography, heterogenous or homogenous microparticle-based ELISA, radioimmunoassay, turbidimetry, nephelometry, ultrasensitive bio-barcode assay or immunoPCR, preferably an ELISA assay, which enables the detection and/or quantification of FusC in a biological sample. The detection and/or quantification steps are conventional steps in this field.

We have unexpectedly found that FusC is present in disease state sera. This is unexpected as FusC has to date been regarded as of secondary importance to TAFC and is actually a biosynthetic intermediate in the TAFC biosynthetic pathway (Haas H., Eisendle M. and Turgeon G. (2008) Siderophores in fungal physiology and virulence. Annu Rev Phytopathol. 46:149-187). Indeed the literature is unambiguous on this point and it is clear that TAFC is expected to be the only, or main, siderophore secreted by A. fumigatus (See Figure 1 in Haas H., Eisendle M. and Turgeon G. (2008) Siderophores in fungal physiology and virulence. Annu Rev Phytopathol. 46: 149-187). Thus, one skilled in the art would not expect an intermediate in a biosynthetic pathway to be secreted in detectable amounts.

Thus, we have unexpectedly found that the presence of FusC, in the biological sample is indicative of infection caused by or associated with siderophore-secreting microorganisms, preferably Aspergillus fumigatus infection, more preferably invasive aspergillosis (IA).

Additionally, we have overcome various problems as outlined below.

The generation of a polyclonal antiserum comprising an anti-FusC antibody is problematic as FusC is a small molecule, molecular weight of 726 Da, with limited epitope structure. Thus, antisera/polclonal antibody generation is not straightforward. We have overcome this problem by synthesising novel immunogens comprising protein-siderophore conjugates.

Competitive ELISA with FusC also presented difficulties in terms of immobilisation of FusC on a solid phase such that it can compete with free FusC for binding to anti-FusC IgG.

We have unexpectedly provided a single competitive ELISA format to detect FusC in biological specimens from multiple species.

We have also unexpectedly provided an anti-FusC antibody or polyclonal antiserum which detects FusC only does not cross-react with TAFC.

We have found that the presence of FusC, in the biological sample is indicative of infection caused by or associated with siderophore-secreting microorganisms, preferably Aspergillus fumigatus infection, more preferably invasive aspergillosis (IA). Optionally, the presence of FusC in a concentration of greater than 3 µg ml, preferably greater than 15 µg ml, in the biological sample is indicative of infection caused by or associated with siderophore-secreting microorganisms.

According to one embodiment of the invention, the method comprises:
providing a solid support having bound thereto FusC or a conjugate thereof;
reacting the FusC or conjugate thereof with an anti-FusC antibody, and a biological sample of a subject; and
detecting and/or quantifying the presence of FusC in the biological sample, wherein the anti-FusC antibody was raised against an immunogen comprising FusC coupled to a carrier protein.

The FusC or conjugate thereof or an anti-FusC antibody, may be labelled directly or indirectly with a detectable label.

The FusC or a conjugate thereof or an anti-FusC antibody, may be labelled with gold nanoparticles.

The solid support may comprise gold nanoparticles.

A method may comprise an initial step of preparing the anti-FusC antibody using a FusC conjugate. Optionally, this step may involve the use of a first and a second FusC conjugate. Each conjugate comprises FusC covalently coupled to a carrier protein, wherein the first FusC conjugate cross-linker chemistry differs to the second FusC conjugate cross-linker chemistry used in the preparation of the FusC antibody. For example, the first FusC conjugate may be KLH-sHSAB- FusC and the second FusC conjugate may be cBSA-SATA-SMCC-FusC. We have recognised that the FusC conjugate used in an assay method could differ to the FusC conjugate used in the generation of the anti-FusC antibody. The FusC conjugate is described in more detail below.

Preferably, the anti-FusC antibody is labelled directly or indirectly with a detectable label.

According to another embodiment of the invention, the method comprises:
(a) providing a solid support having bound thereto an anti-FusC antibody;
(b) reacting the anti-FusC antibody with FusC or a conjugate thereof, and a biological sample of a subject; and
(c) detecting and/or quantifying the presence of FusC in the biological sample, wherein the anti-FusC antibody was raised against an immunogen comprising FusC covalently coupled to a carrier protein.

Preferably, the FusC or conjugate thereof is labelled directly or indirectly with a detectable label.

Preferably, the anti-FusC antibody is labelled directly or indirectly with a detectable label.

As used herein, the term "conjugate" is intended to mean a siderophore (hapten) such as FusC, covalently linked to another moiety, preferably a carrier protein. According to the invention, these are protein-FusC conjugates, which we have unexpectedly found may be used both as immunogens or as ELISA antigens.

These protein FusCconjugates may be used as immunoassay antigens and/or in the generation of anti-siderophore antibodies. We have unexpectedly found that the immunogens which led to the generation of anti-FusC antibodies were made by conjugating the FusC hapten (FusC) to a carrier protein.

Optionally, the carrier protein may be keyhole limpet haemocyanin (KLH) or bovine serum albumin (BSA), preferably cationised BSA (cBSA).

Optionally, the siderophore may have chelated Fe³⁺.

These conjugates may be formed in different ways. For example, conjugates may be prepared using either KLH or BSA as the protein carrier and via sHSAB-mediated hapten coupling. Alternatively, conjugates may be prepared using cationised BSA (cBSA) only, as protein carrier, and hapten conjugation was by thioether coupling.

During the conjugation of the hapten to the protein, we found it was essential (i) not to interfere with epitope availability on the hapten and, (ii) to avoid subsequent antibody cross-reactivity with either the chemical linker or linker-hapten moiety. FusC is a small molecule with limited epitope availability. We achieved conjugation of FusC to carrier proteins and avoided conjugate polymerisation and epitope occlusion. This was monitored by the ability of free FusC to prevent IgG [anti-FusC] binding to immobilised FusC. One conjugation method involved covalently attaching a photoactivatable cross-linker to a carrier protein, followed by addition and subsequent covalent attachment of the siderophore to the activated carrier protein, by UV exposure. This immunogen was functional and detected bound
siderophores, and induced FusC antibody production upon immunisation. Another conjugation method involved separate activation of the carrier protein and FusC, respectively, by covalent modification, following by mixing and thioether bond formation leading to stable and functional immunogens.

Suitable conjugates of FusC include, but are not limited to, KLH-sHSAB-FusC, BSA-sHSAB-FusC, cBSA-SATA-SMCC-FusC, cBSA-SATA-SMCC-FusC(Fe³⁺), and combinations thereof; wherein BSA is bovine serum albumin, cBSA is cationised bovine serum albumin, KLH is keyhole limpet haemocyanin, sHSAB is N-Hydroxysulfosuccinimidyl-4-azidobenzoate available from Thermo Fisher Scientific, Northumberland, UK, SATA is N-succinimidyl-S-acetylthioacetate available from Thermo Fisher Scientific, Northumberland, UK, and SMCC is succinimidyl 4-[N-maleimidomethyl]cyclohexane-1-carboxylate available from Thermo Fisher Scientific, Northumberland, UK. Optionally, the conjugate, when present, is cBSA-SATA-SMCC-FusC(Fe³⁺) or a combination thereof.

According to a preferred embodiment of the invention, the conjugates may be selected from the group comprising KLH-sHSAB-FusC, cBSA-SATA-SMCC-FusC and cBSA-SATA-SMCC-FusC(Fe³). Ideally, the immunogen is KLH-sHSAB-FusC and the ELISA antigen is cBSA-SATA-SMCC-FusC or cBSA-SATA-SMCC-FusC(Fe³⁺).

Importantly, these FusC conjugates may be used as both as immunogens, in the generation of polyclonal antisera and antibodies, and as immunoassay antigens, for example as ELISA antigens.

As used herein, the term "detectable label" refers to a molecule which may be attached covalently or non-covalently to a moiety and which permits analysis of the moiety.

Suitable detectable labels include or comprise those which permit analysis by flow cytometry, e.g. fluorescein isothiocyanate, and those useful in colorimetric enzyme systems, e.g., horseradish peroxidase (HRP) and alkaline phosphatase (AP). Other suitable detectable labels include or comprise gold or silver nanoparticles, latex nanoparticles, silica microparticles or quantum dots. It will be appreciated that any other suitable detectable label not included herein may be used.

In all of the methods outlined in the specification, the FusC or conjugate thereof or anti-FusC antibody may be labelled directly or indirectly with a detectable label.

Optionally, the detectable label may comprise gold nanoparticles.

Gold nanoparticles are stable labels, or surfaces, onto which proteins can be immobilised. As labels, they provide a high sensitivity of detection for biological analytes and their use avoids the requirement for expensive equipment as they can be visually detected. Gold nanoparticles can also be used as labels for lateral flow immunoassays (Posthuma-Trumpie G.A., Korf J. and van Amerongen A. (2009) Lateral flow (immuno)assay: its strengths, weaknesses, opportunities and threats. A literature survey. Anal Bioanal Chem 393:569-582).

The use of gold nanoparticles may provide for the use of homogenous immunoassays, and also provide a method for point-of-care immunoassays, thereby complementing the requirement for laboratory-based testing.

Furthermore, the solid support may comprise gold nanoparticles. Conventionally, the solid support on which immunoassays is performed is composed of plastic. The ability to immobilise FusC on spherical beads like gold nanoparticles enables the formulation of a homogenous competitive assay using free IgG competing for binding to either immobilised fusC or free FusC. Alternatively, enzyme or gold-labelled -FusC could compete with free FusC for binding to immobilised IgG.

Optionally, the detectable label may comprise HRP. When the detectable label comprises HRP, the method optionally further comprises providing a substrate for HRP, e.g. tetramethylbenzidine, TMB. It will be appreciated that the term "labelled directly" means that the detectable label is bound to the anti-siderophore antibody or siderophore or conjugate thereof to be detected, as appropriate. It will be appreciated that the term "labelled indirectly" means that the detectable label comprises an entity capable of binding to the anti-siderophore antibody or siderophore or conjugate thereof to be detected, as appropriate, wherein the entity is preferably an anti-species antibody. Optionally, when the anti-siderophore antibody or siderophore or conjugate thereof, as appropriate, is labelled indirectly, the detectable label comprises an anti-species IgG-HRP conjugate, optionally an IgG[anti-rabbit IgG]-horseradish peroxidase conjugate.

The solid support may comprise a plastics material, glass, nitrocellulose or metal. Alternatively, the solid support may comprise a combination of a plastics material or glass or nitrocellulose, with metal, such as metal-coated plastics material or metal-coated glass. Suitable plastics materials include polymers such as polystyrene, polyethylene, polypropylene, polytetrafluoroethylene, polyamide, polyacrylamide and polyvinylchloride, optionally in microparticulate form. Polystyrenes sold under the trade name Nunc Maxisorp™ (Nunc A/S, Roskilde, Denmark) are preferred. Suitable metals include gold, silver, aluminium, chromium and titanium, optionally in microparticulate or nanoparticulate form.

The biological sample is preferably a biological fluid selected from whole blood, blood plasma, blood serum, urine, saliva, bronchoalveolar lavage, perspiration and tears, more preferably selected from urine, whole blood, blood plasma and blood serum. Blood serum, blood plasma or urine is especially preferred.

The subject may be a human or non-human animal, preferably mammalian. Non-human animals include but are not limited to avian, equine, canine, feline, bovine, caprine, porcine, ovine and rodent, including murine, species, preferably avian, canine and murine species. Humans are especially preferred.

Optionally, the biological fluid may be subjected to a dilution from a 1/5 to a 1/15 dilution, preferably a 1/10 dilution, before use in the method.

Optionally, the method is conducted within 10 days of the subject being infected by an infection caused by or associated with FusC-secreting microorganisms, preferably within 5 days of the subject being infected.

Advantageously, the presence of FusC is indicative of an infection caused by or associated with FusC-secreting microorganisms in the subject.

Optionally, the presence of FusC in a concentration of greater than 3 µg/ml in the biological sample is indicative of infection caused by or associated with FusC-secreting microorganisms in non-human animals.

Optionally, the presence of FusC in a concentration of greater than 15 µg/ml in the biological sample is indicative of infection caused by or associated with FusC-secreting microorganisms in humans.

Optionally, the method is performed in accordance with an immunoassay selected from the group comprising enzyme-linked immunosorbent assay (ELISA), immunochromatography, heterogenous or homogenous microparticle-based ELISA, radioimmunoassay, turbidimetry, nephelometry, ultrasensitive bio-barcode assay or immunoPCR, preferably ELISA.

According to another embodiment of the invention, the method comprises the use of an antibody raised against an immunogen comprising FusC covalently coupled to a carrier protein.

Immunisation with free FusC does not tend to stimulate antibody production. We speculate that this is because these molecules are less than 1000 Da molecular mass. We have found that conjugation of the siderophore to carrier proteins is required prior to immunisation. Polyclonal antisera raised against FusC was obtained from rabbits following immunisation with the FusC-protein conjugates of the invention. We have found that the optimal titre, or dilution, for antisera use in ELISA ranged from approximately
1/500 - 1/2000. No reactivity against FusC was detectable in pre-immune antisera and, importantly, provided for elimination of any potential cross-reactivity with either the carrier protein or cross-linker used for antibody generation.

The antibody may be a polyclonal antibody, optionally present in polyclonal antisera derived from a human or non-human animal presented with the immunogen. Optionally, the polyclonal antibody is an isolated or purified polyclonal antibody.

Alternatively, the antibody may be a monoclonal antibody derived using techniques known in the art.

Preferably, the carrier protein is keyhole limpet haemocyanin (KLH) or bovine serum albumin (BSA), preferably cationised BSA (cBSA).

Optionally, the FusC may have chelated Fe³⁺.

According to a preferred embodiment, the antibody may be raised against an immunogen selected from KLH-sHSAB-FusC, BSA-sHSAB-FusC, cBSA-SATA-SMCC-FusC and cBSA-SATA-SMCC-FusC(Fe³⁺); wherein BSA is bovine serum albumin, cBSA is cationised bovine serum albumin, KLH is keyhole limpet haemocyanin, sHSAB is A/-Hydroxysulfosuccinimidyl-4-azidobenzoate available from Thermo Fisher Scientific, Northumberland, UK, SATA is N-succinimidyl-S-acetylthioacetate available from Thermo Fisher Scientific, Northumberland, UK, and SMCC is succinimidyl 4-[N-maleimidomethyl]cyclohexane-1-carboxylate available from Thermo Fisher Scientific, Northumberland, UK.

According to a more preferred embodiment, the antibody may be raised against an immunogen selected from the group comprising KLH-sHSAB-FusC, cBSA-SATA-SMCC-FusC and cBSA-SATA-SMCC-FusC(Fe³⁺). However, it will be appreciated that any other suitable immunogen be used. Suitable immunogens ideally consist of carrier protein covalently coupled FusC, presented in an antigenic form and which induce antibody production, said antibodies should then be capable of recognising free FusC.

Optionally, the antibody according may be obtainable by the following method comprising steps
(i) immunizing an animal, preferably a non-human animal, with at least one immunogen selected from KLH-sHSAB-FusC, BSA- sHSAB-FusC, cBSA-SATA-SMCC-FusC, cBSA-SATA-SMCC-FusC(Fe³⁺), preferably KLH-sHSAB-FusC, cBSA-SATA-SMCC-FusC and cBSA-SATA-SMCC-FusC(Fe³⁺); and
(ii) isolating polyclonal antiserum from said animal.

According to another embodiment of the invention, the antibody, referred to as an anti-FusC antibody, is capable of recognizing ferrated and non-ferrated FusC, does not bind to TAFC and has an IC₅₀ = 0.4 µg ml.

Optionally, the anti-FusC antibody is present in a FusC polyclonal antiserum, preferably wherein the FusC polyclonal antiserum comprises anti-KLH-sHSAB-FusC.

According to another general aspect of the invention, there is provided a kit for carrying out the method of the invention.

According to one embodiment, there is provided a kit for use in detecting fusarinine C (FusC) and/or detecting infections caused by or associated with FusC -secreting microorganisms, preferably an Aspergillus fumigatus infection, more preferably invasive aspergillosis (IA), in a biological sample of a subject comprising:
a. a solid support having bound FusC or a conjugate thereof; and
b. an anti- FusC antibody, wherein the anti-FusC antibody was raised against an immunogen comprising FusC covalently coupled to a carrier protein.

According to another embodiment, the kit comprises:
a. a solid support having bound an anti-FusC antibody, wherein the anti-FusC antibody was raised against an immunogen comprising FusC covalently coupled to a carrier protein; and
b. FusC or a conjugate thereof.

Optionally, the anti-FusC antibody or FusC or a conjugate thereof may be labelled directly or indirectly with a detectable label, preferably gold nanoparticles. The detectable label, when present, is preferably as defined above.

Optionally, in all of these embodiments the detectable label or the solid support may comprise gold nanoparticles.

It will be understood that the kit of the invention may comprise at least one antibody as defined above.

Advantages of the invention include:
- The siderophore FusC may be directly immobilised on the solid support, or, alternatively, indirectly immobilized when included as a conjugate, depending on the preferred mode of operation.
- The assay provides a simple, accurate and cost-efficient method for detecting infections caused by or associated with FusC -secreting microorganisms, preferably an Aspergillus fumigatus infection, which has not previously been demonstrated.

The invention will now be described by the following non-limiting figures and examples.
**Figure 1****:** Structures of various reagents used for antisera generation and ELISA analysis.

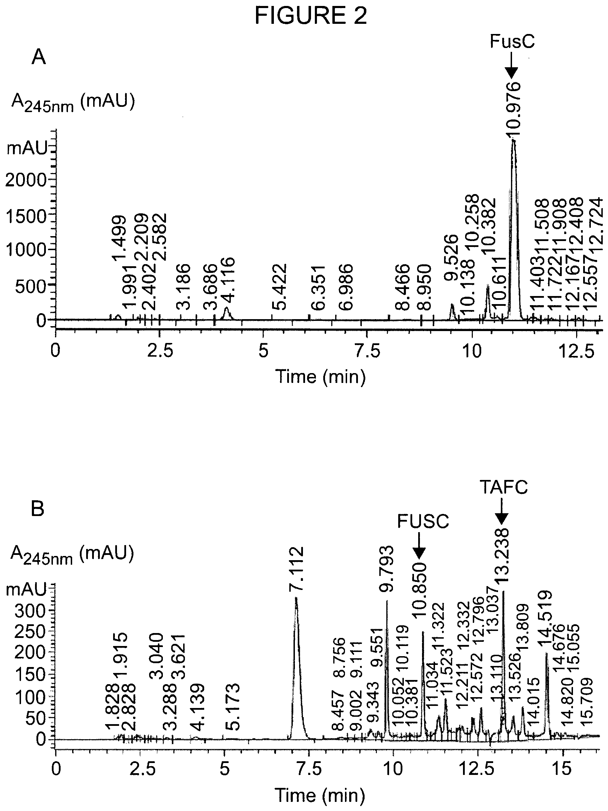

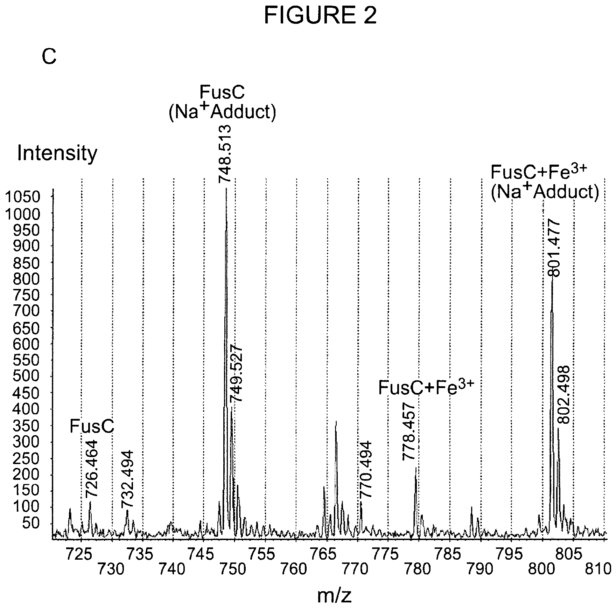

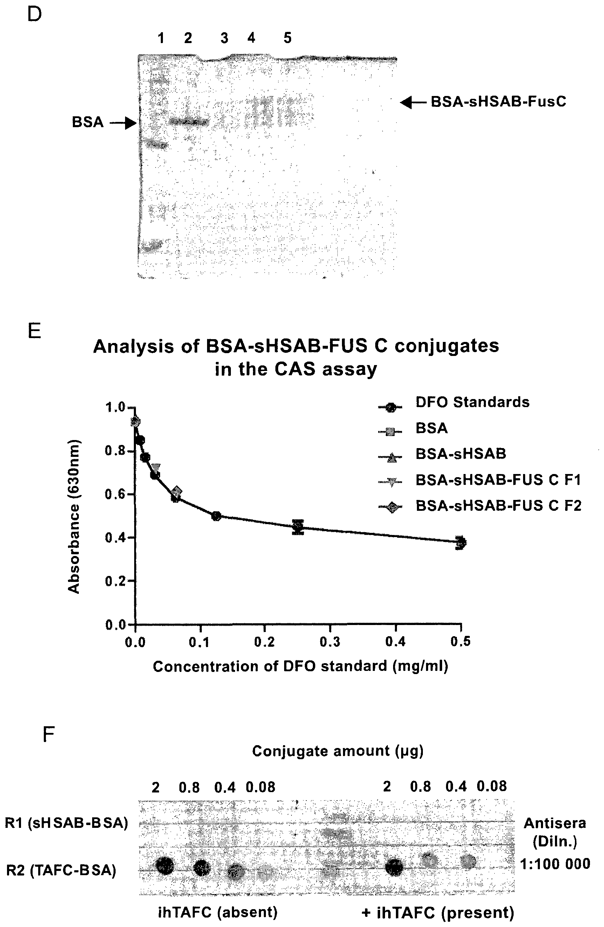

**Figure 2****:** Preparation and analysis of BSA-sHSAB-FusC or ihTAFC conjugates (PSC1). **A.** RP-HPLC confirmation of FusC purity. **B.** RP-HPLC confirmation of ihTAFC which contained FusC and TAFC. **C.** MALDI-ToF mass spectrometric analysis of purified FusC. H+ and Na+ adducts, showed the expected masses of ferrated and non-ferrated forms of FusC (Table 2). **D.** SDS-PAGE analysis of BSA, sHSAB-activated BSA and two BSA-sHSAB-FusC (PSC1D) conjugates prepared using two different molar excesses of FusC. L1 = Mr ladder, L2 = BSA, L3 = BSA-sHSAB (UV treated), L4 = BSA-sHSAB-FusC (PSC1D; F1), L5 = BSA-sHSAB-FusC (PSC1D; F2). Different amounts of FusC were present in immunogen conjugation reactions for F1 (0.4 mg) and F2 (1.2 mg), respectively. A 220 molar excess was used as per the ihTAFC conjugation method. L5 - F2 shows a shift in molecular mass which is not seen in L4 with F1. No Molecular weight shift has been clearly seen before only a shift in the smeared band. **E.** Chrome Azurol S analysis of FusC conjugation to BSA (lower absorbances observed for conjugates F1 and F2 confirm successful siderophore conjugation). Conjugate F2 is shown to contain more FusC than F1, as expected, since there was more FusC present in the conjugation reaction. **F.** Immunoblot analysis of murine anti-TAFC reactivity. Immobilised protein conjugates (R1 (sHSAB-BSA) or R2 (ihTAFC-sHSAB-BSA; PSC1C)) were present at 2, 0.8, 0.4 and 0.08 µg, respectively. No reactivity towards HSAB-BSA is evident, while TAFC is recognised when bound to protein (R2). Inhibition of antibody binding is evident due to free TAFC presence, especially at 0.08 µg ihTAFC-sHSAB-BSA (PSC1C). (ihTAFC: in-house TAFC).

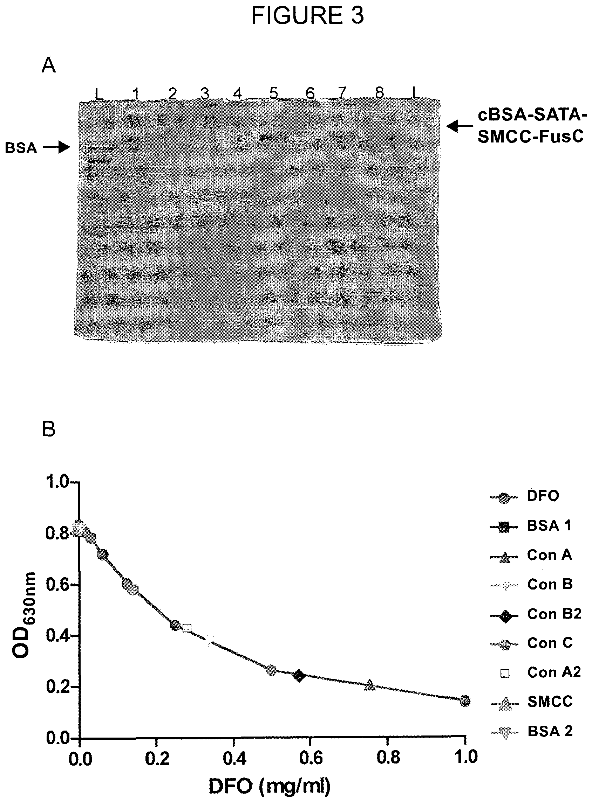

**Figure 3****:** Characterisation of PSC2A&3A conjugates (cBSA-SATA-SMCC-FusC). **A.** SDS-PAGE analysis. Conjugate A = Acetylated PSC3A (Activated & acetylated FusC:SATA-cBSA, 13.5:5.66 mg); Conjugate B = PSC2A (Activated FusC:SATA-cBSA, 13.5:5.66 mg); Conjugate C = *PSC2A* (Activated FusC:SATA-cBSA, 0.5:0.21 mg). Key: Lane 1: Conjugate A, 2: Conjugate B, 3: Conjugate B2, 4: Conjugate C, 5: SATA-cBSA, 6: Conjugate A2, 7: SMCC: SATA-cBSA, 8: SATA-cBSA, L: Mr markers. (protein: 2 µg per well). Note the appearance of conjugate, only when FusC is present (A, A2, B, B2 and C). **B.** Estimation of conjugated FusC in *PSC2&3* conjugates by CAS analysis. Plot of OD_{630 nm} versus [DFO] mg/ml. Conjugates A, B and C contained 753, 336 and 140 µg/ml DFO equivalents. (Note: Conjugates A2 (280 µg/ml DFO equivalents) and Conjugate B2 (570 µg/ml DFO equivalents) were from previous syntheses. No colour change (i.e., FusC siderophore presence) was detectable for BSA, SATA-BSA or SMCC-SATA-BSA (Table 3). These data confirm that FusC is covalently coupled to BSA and that Fe³⁺⁻binding affinity is intact.

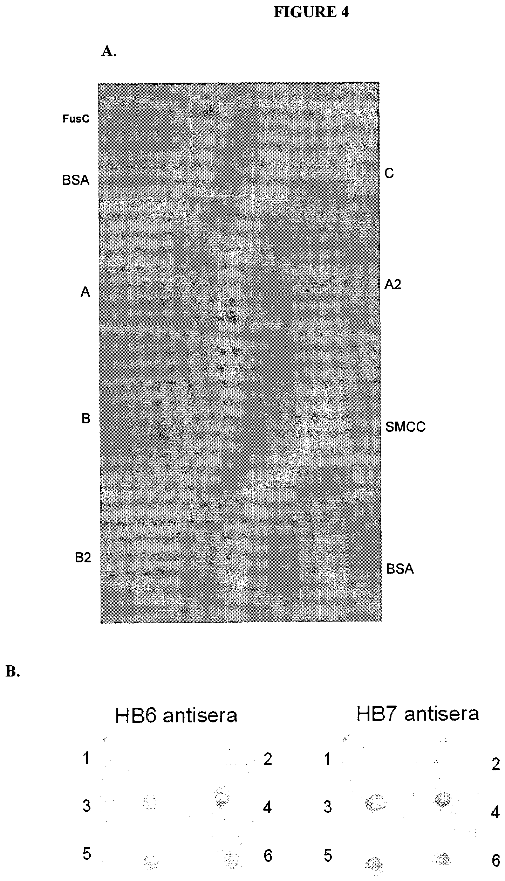

**Figure 4****:** Analysis of *PSC2* & 3 (cBSA-SATA-SMCC-FusC) conjugates. **A.** Direct analysis of *PSC2* & 3 conjugates (see legend to Figure 3 for conjugate identity) by Fe³⁺ chelation. FusC (10 µg), along with 10 µg FusC equivalents of *PSC2* & 3 conjugates were applied to nitrocellulose and allowed to dry. The blot was then submerged in iron sulphate for 5 min and removed. The iron binding to FusC was visualised by a red-brown colour where the FusC or conjugates were present. **B.** Immunoblot analysis of *PSC2* and *PSC3,* and related conjugates using rabbit antisera (*n* = 2 (termed HB6 & 7); raised against KLH-sHSAB-ihTAFC (*PSC1A*). Key: Antigen (Ag) 1: cBSA-SATA; Ag2: cBSA-SMCC; Ag3: cBSA-FusC (acetylated) (*PSC3*); Ag4: cBSA-SATA-SMCC-FusC (non-acetylated) (*PSC2*); Ag5: cBSA-SATA-SMCC-FusC (mix of non-acetylated and acetylated) (*PSC2*&*3*); Ag6: cBSA-SATA-SMCC-FusC (mix of non-acetylated and acetylated + Fe³⁺) (*PSC2B*&*3B*). No reactivity was observed against SATA- or SMCC-modified cationised-BSA. Antibody reactivity is observed against *PSC2,* and equivalent reactivity is observed +/-acetylation or +/-Fe³⁺ presence.

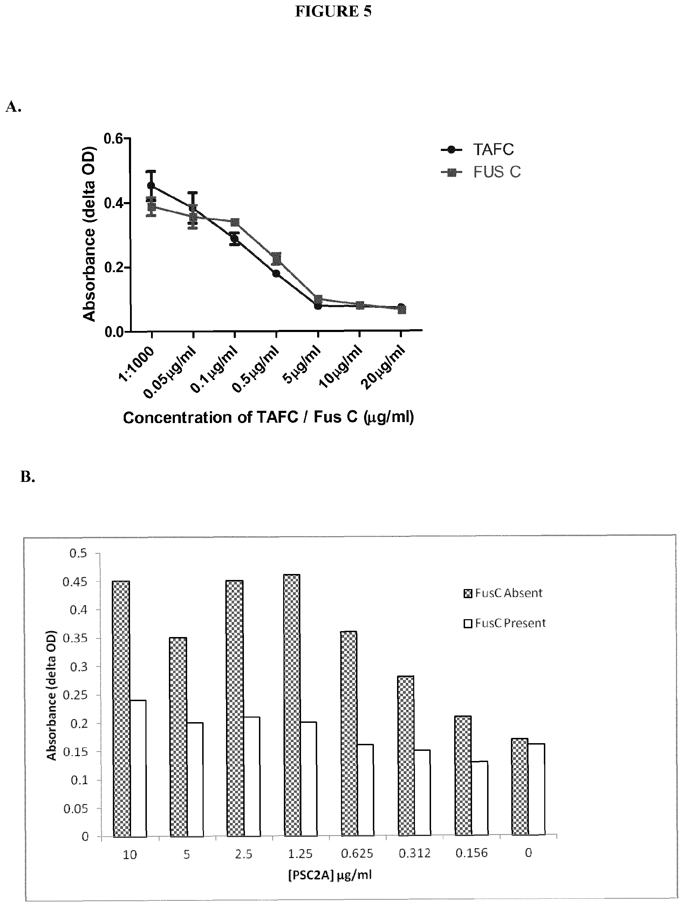

**Figure 5****:** Competitive ELISA detection of TAFC and FusC. **A.** Immobilised antigen was an equimolar combination of *PSC2B and PSC3B* (acetylated & non-acetylated cBSA-SMCC-FusC^{Fe3+}), coated at 2.5 µg/ml (250 ng/well). Murine antisera was used (1/1000) which was raised against KLH-sHSAB-ih/cTAFC (*PSC1A* & *PSC1B*); Competition antigens were either FusC or cTAFC (0 - 20 µg/ml, respectively). **B.** FusC detection by ELISA. Free FusC 6.5 µg/ml prevents antibody (anti-*PSC1A*, rabbit antisera) recognition of conjugate *PSC2A* (cBSA-SATA-SMCC-FusC).

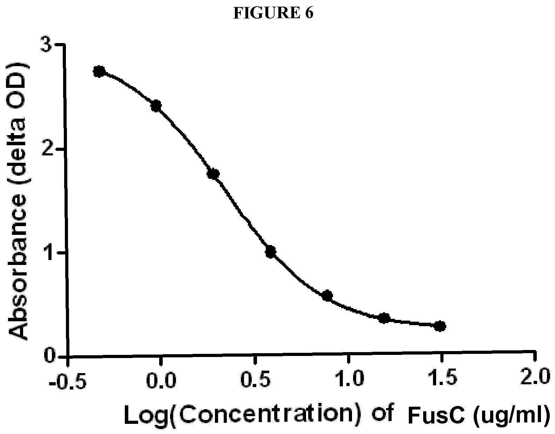

**Figure 6****:** Standard curve obtained for FusC detection by competitive ELISA (Formats EF2 or EF3, respectively). Plot of Absorbance (A_{450/630nm} versus log [FusC], 0-100 µg/ml).

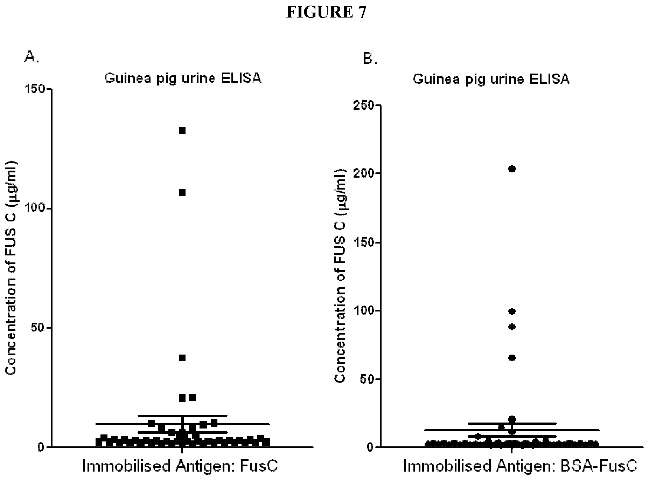

**Figure 7****:** ELISA detection of FusC in urine of animal model (Guinea pig) of invasive pulmonary aspergillosis. Elevated FusC levels were evident in urine whether **A.** Immobilised FusC or **B.** Immobilised *PSC2B* & *PSC3B* (combined) is used in the ELISA. Immobilised antigen concentrations: FusC (16 µg/ml) and *PSC2B* & *PSC3B* (combined) (10 µg/ml). Rabbit antisera (HB7) dilution: 1/1000. Animals were grouped according to the following categories: (i) uninfected controls (*n* = 3 per day), (ii) immunocompromised but *A. fumigatus*-uninfected controls (*n* = 3 per day) (iii) immunocompromised & *A. fumigatus-*infected animals (*n* = 8 per day). Specimens were collected on Day 0, 5, 7 and 9 post-infection or experiment initiation.

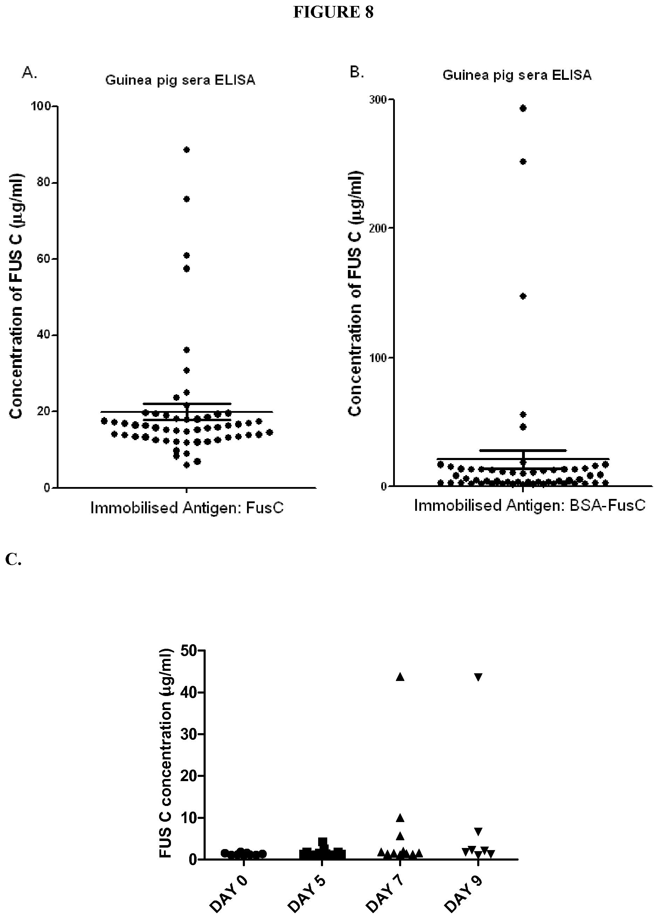

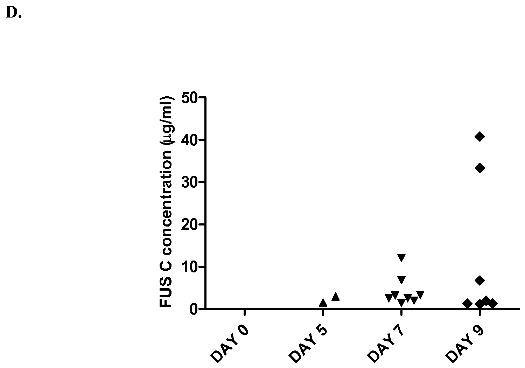

**Figure 8****:** ELISA detection of FusC in sera of animal model of invasive pulmonary aspergillosis. Elevated FusC levels were evident in sera whether **A.** immobilised FusC or **B.** immobilised *PSC2B* & *PSC3B* (combined) was used in the ELISA. Immobilised antigen concentrations: FusC (16 µg/ml) and *PSC2B* & *PSC3B* (combined) (10 µg/ml). Final antisera (HB7) dilution: 1/1000. **C.** Urine samples from animal model assessed by EF3 ELISA. FusC was detectable in some samples as early as day 5 post-infection. **D.** Urine samples from animal model assessed by EF2 ELISA. FusC was detectable in some samples as early as day 5 post-infection. Animals were grouped according to the following categories: (i) uninfected controls (*n* = 3 per day), (ii) immunocompromised but *A. fumigatus-*uninfected controls (*n* = 3 per day) (iii) immunocompromised & *A. fumigatus-infected* animals (*n* = 8 per day). Specimens were collected on Day 0, 5, 7 and 9 post-infection or experiment initiation.

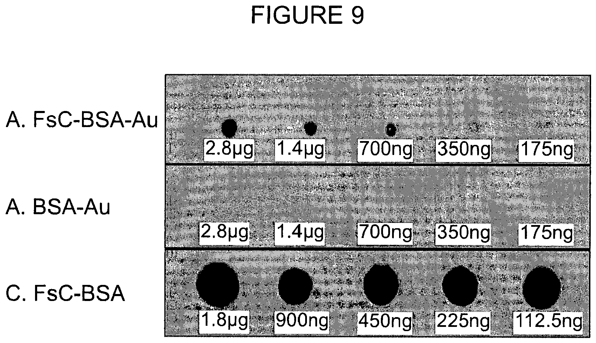

**Figure 9****:** Protein immunoblot of FusC-BSA-AuNP (A), BSA-AuNP (B) and FusC-BSA (C). High reactivity against FusC is clearly evident for the FusC-BSA conjugate across all concentrations. Reactivity is also visible for the FusC-BSA-AuNP conjugate - confirming the conjugation of FusC-BSA to gold nanoparticles. No reactivity is visible for the BSA-AuNP control.

The following examples serve to illustrate the invention but it will be appreciated that the invention is not limited to these examples.

### EXAMPLES

### EXAMPLE 1

### METHODS

### 1. Fusarinine C (FusC) and Triacetylfusarinine C (TAFC) purification.

*Aspergillus fumigatus* ATCC 46645 was cultured for 72 hr under iron-free conditions to induce siderophore secretion. All glassware was treated with 1 mM EDTA for 16 hr and 6 M HCI for approximately 24 hr to ensure that all traces of iron were removed from the glassware. Specifically, *A. fumigatus* cultures were grown in a mineral salt medium (pH 6.8) composed of 25 g/l glucose, 3.5 g/l (NH₄)₂SO₄, 2.0 g/l KH₂PO₄, 0.5 g/l MgSO₄, 8 mg/l ZnSO₄. The pH of the media was brought to 6.8 with 6 M NaOH before autoclaving. Medium (500 ml) containing no iron, in 1 L flasks, was inoculated with *A. fumigatus* conidia at a final concentration of 10⁷ per ml and grown at 37°C in a shaking incubator at 230 rpm, for 72 hr. FusC was purified from culture supernatants by passage through Sep-Pak C₁₈ cartridges (Waters Limited, Herts, UK), removal of unbound material using deionised water and elution of bound FusC in methanol prior to vacuum concentration to dryness. FusC purity was assessed by RP-HPLC and identity confirmed by MALDI-ToF MS. MALDI-ToF MS was carried out using an Ettan™ MALDI-ToF mass spectrometer (Amersham). FusC was deposited with 1 µl α-cyano-4-hydroxycinnaminic acid onto mass spectrometry slides and allowed to dry prior to delayed extraction, reflectron ToF analysis at 20 kV. Crude TAFC was prepared as follows: Acidified culture supernatant (28 ml; pH 2.75) was passed through an Amberlite XAD-2 column (Sigma-Aldrich, Poole, UK) (bed volume: 7 ml) and the flow- through discarded. The column was then washed with 5 bed volumes of deionised H₂O to remove all unbound components. The column was then eluted with 20 ml of 50% (v/v) methanol to displace bound siderophores. The eluate was collected and evaporated to dryness under vacuum in rotary vacuum evaporator. This preparation also contained FusC and is herewith referred to as in-house TAFC (ihTAFC). Pure TAFC was obtained on a commercial basis, termed cTAFC (EMC Microcollections). A commercially available Chrome Azurol S (CAS) assay (SideroTec Assay™, www.emergenbio.com) was used to determine FusC and TAFC presence and to assess if conjugation to proteins, to make immunogens, was successful. Briefly, equal volumes (100 µl) of CAS reagent and Desferrioxamine (DFO; a siderophore) standards, and samples, (in duplicate) were added to microwells and allowed to react for 15 min followed by absorbance measurement at 630 nm. Standard curves were prepared by plotting A_{630 nm} versus [DFO] mg/ml.

### 2. Immunogen synthesis and antibody generation.

Cationised BSA (cBSA) synthesis: Ethylenediamine dihydrochloride (EDA) and hexamethylenediamine dihydrochloride (HEX) were used to activate BSA and introduce extra amino (-NH₂) groups for hapten conjugation. EDA (10 ml; 0.1 M) was added to 10 ml BSA (5 mg/ml in Phosphate Buffered Saline (PBS)) followed by 1-Ethyl-3-(3-dimethylaminopropyl) carbodiimide (EDC) (40 mg). The reaction mixture was stirred at room temperature for 2 hr and then dialysed into PBS, 3 times (3 hr, overnight and 3 hr) with stirring, at 4 °C. HEX activation of BSA was performed identically to also yield cationised BSA (cBSA) where the majority of carboxyl groups were converted to -NH₂ groups.

*Protein-siderophore conjugate 1 (PSC1):* BSA (3 mg/900 µl) and *N*-Hydroxysulfosuccinimidyl-4-azidobenzoate (sHSAB (Thermo Fisher Scientific, Northumberland, UK)) (3 mg/1.5 ml) were reacted together for 1 hr at room temperature in the dark prior to conjugation to ihTAFC or cTAFC. This represented a 184 molar excess of sHSAB over BSA. Unbound sHSAB was removed by gel filtration using a Sephadex G-25 PD10 column in the dark - equilibrated with PBS whereby activated sHSAB-BSA was eluted in 3.5 ml PBS (final concentration: 0.857 mg/ml). This solution was adjusted to BSA-sHSAB (0.5 mg/ml final) in PBS. Either 1.2 mg of ihTAFC or cTAFC in 50 µl of PBS was added to 0.5 mg/ml of sHSAB-BSA (giving a 220 molar excess) in bijou tubes. Bijous were placed on ice and UV-irradiated at 302 nm for 10 min using a UV lamp at a distance of 5 cm. Keyhole Limpet Haemocyanin (KLH) was also likewise activated by sHSAB for conjugation to ihTAFC (TAFC/Fus C) or cTAFC. KLH (3 mg) = 0.3 nmol, sHSAB (3 mg) = 8287 nmol therefore there was a 27623 molar excess of sHSAB over KLH. sHSAB-KLH was reacted with 1 mg of cTAFC or ihTAFC, respectively, as per sHSAB-BSA reactions. Putative conjugates were dialysed overnight at 4 °C into PBS to remove unwanted reactants. Analysis of conjugates was undertaken by CAS assay, colour change following iron addition and SDS-PAGE.

*Protein-siderophore conjugate 2 (PSC2):* N-succinimidyl-S-acetylthioacetate (SATA; Thermo Fisher Scientific, Northumberland, UK) was used to introduce blocked thiol groups onto cationised BSA (cBSA) and this activated protein was termed SATA-cBSA (Fox M, Gray G, Kavanagh K, Lewis C, Doyle S. (2004) Detection of Aspergillus fumigatus mycotoxins: immunogen synthesis and immunoassay development. J Microbiol Methods. 56(2):221-230). Purified FusC was activated with limiting amounts of succinimidyl 4-[N-maleimidomethyl]cyclohexane-1-carboxylate (SMCC; Thermo Fisher Scientific, Northumberland, UK) at SMCC:FusC molar ratio of 0.49 (SMCC:NH₂ group ratio: 0.16) for 2 hr at room temperature. After exposing thiol groups on SATA-cBSA (i.e., demasking using 1/10 vol 0.5 M hydroxylamine addition for 60 min), SMCC-activated FusC was added to this solution, followed by reaction for 30 min at room temperature. After reaction, conjugates were dialysed 3 times (3 hr, overnight and 3 hr) with stirring, at 4 °C.

*Protein-siderophore conjugate (PSC3):* Chemically acetylated FusC conjugated to cBSA. Chemical acetylation of SMCC-activated FusC, to yield SMCC-activated TAFC, was undertaken as follows, prior to conjugation to SATA-cBSA. Acetic anhydride (53 µl) was added to 1.5 ml 1 M Na₂HPO₄. This solution was then added in two 750 µl aliquots, at T = 0 and 30 min to SMCC-activated FusC (containing 13.5 mg FusC) and incubated on ice a further 30 min. The conjugate was transferred to room temperature before addition to demasked SATA-cBSA (3.13 nmol; 209.7 µg).

A full list of the immunogens (protein-sideophore conjugates) used is provided in Table 1.

**Table 1: Identity of all conjugates used for FusC and TAFC antisera generation and ELISA, respectively. PSC1 conjugates were prepared using either KLH or BSA as the protein carrier and via sHSAB-mediated hapten coupling. PSC2 and PSC3 conjugates were prepared using cationised BSA (cBSA) only, as protein carrier, and hapten conjugation was by thioether coupling. PSC3 conjugates were prepared using acetylated FusC.**

| **Conjugate Acronym** | **Conjugate** |
|---|---|
| | **Composition** |
| *PSC1* | (A) KLH-sHSAB-ihTAFC; (B) KLH-sHSAB-cTAFC; (C) BSA-sHSAB-ihTAFC; (D) BSA-sHSAB-FusC. |
| *PSC2* | (A) cBSA-SATA-SMCC-FusC; (B) cBSA-SATA-SMCC-FusC(Fe³⁺) |
| *PSC3* | (A) cBSA-SATA-SMCC-Acetylated FusC; (B) cBSA-SATA-SMCC-Acetylated FusC(Fe³⁺) |

Animals, New Zealand White rabbits and mice, respectively, were immunised with *PSC1A&B;* (rabbits (*PSC1A* only) and mice (*PSC1A&B*)) or *PSC2A&B* (rabbits only) over the following schedule (Day 0, immunisation; Day 14, boost 1; Day 28, boost 2: Day 42, boost 3: Day 56, boost 4: Day 70, boost 5: Day 77 exsanguination). Antisera, either test or final bleeds, was collected at stored at -20 °C until required for use.

### 3. FusC ELISA - Optimisation

Microtitre plates (Nunc Maxisorp™ available from Nunc A/S, Roskilde, Denmark) were coated with either FusC (range used for optimisation: 0-66 µg/ml) or an equimolar combination of *PSC2B*/*PSC3B* conjugates (range used for optimisation: 0-10 µg/ml) in 50 mM sodium carbonate pH 9.4 for 16 hr (100 µl/well). Then, excess coating reagents (either excess unbound FusC or *PSC2B*/*PSC3B* conjugates in 50 mM sodium carbonate pH 9.4) were removed and microtitre plates were then blocked with 1% (w/v) BSA and 10% (w/v) sucrose in 50 mM sodium carbonate pH 9.4 (200 µl/well) for 2 hr at 37 °C, after which the blocking agent was removed and microplates dried at 37 °C overnight. To commence ELISA analysis, TAFC or FusC calibrators (50 µl each; 0- 100 µg/ml), or diluted specimens (50 µl) were (i) added to microplates, followed by equivalent volumes of rabbit antisera [anti-FusC] (diluted 1/100-1/100,000 in PBST), or (ii) co-incubated with equivalent volumes of rabbit antisera [anti-FusC] (diluted 1/100-1/100,000 in PBST) for 1 hr, prior to addition to microplates. (Note: on occasion TAFC or FusC calibrator range was 0-20 µg/ml). Following addition, and incubation on microplates at room temperature (18-22 °C) for 1 hr, reagents were removed by Phosphate Buffered Saline-0.05%(v/v) Tween-20® (PBST) washing (4x), followed by addition of IgG[anti-rabbit IgG]-horseradish peroxidase conjugate (1/1000 dilution; 100 µl/well) for 1 hr. After conjugate removal and PBST washing (4x), Tetramethylbenzidine (TMB) substrate (100 µl/well) was added for 15 min and the reaction terminated by addition of 1N H₂SO₄ (100 µl/well). Finally the absorbance was measured at 450/620 nm using a Synergy platereader and standard curves of A_{450/620nm} versus log FusC concentration (0-100 µg/ml (or 0-20 µg/ml TAFC) constructed, from which the concentration of FusC in normal clinical specimens, of animal and human origin, was computed.

### 4. Clinical specimen analysis and ELISA validation

Clinical specimens were obtained from (i) a Guinea pig animal model of invasive pulmonary aspergillosis and control specimens (urine and plasma; both control and disease-state specimens), (ii) normal human sera and (iii) disease-state sera from individual patients (*n* = 3) with invasive aspergillosis. All specimens were assayed at an optimal dilution (1/10-1/40) in the FusC ELISA, optimised in section 3 above, as follows.

Microtitre plates (Nunc Maxisorp™ available from Nunc A/S, Roskilde, Denmark) were coated with either FusC (16 µg/ml) or an equimolar combination of *PSC2B*/*PSC3B* conjugates (10 µg/ml) in 50 mM sodium carbonate pH 9.4 for 16 hr (100 µl/well). Then, excess coating reagents (either excess unbound FusC or *PSC2B*/*PSC3B* conjugates in 50 mM sodium carbonate pH 9.4) were removed. Microtitre plates were then blocked with 1% (w/v) BSA and 10% (w/v) sucrose in 50 mM sodium carbonate pH 9.4 (200 µl/well) for 2 hr at 37 °C, after which the blocking agent was removed and microplates dried at 37 °C overnight. To commence ELISA analysis, TAFC or FusC calibrators (50 µl each; 0- 100 µg/ml), or diluted specimens (50 µl) were (i) added to microplates, followed by equivalent volumes of rabbit antisera [anti-FusC] (diluted 1/500 in PBST), or (ii) co-incubated with equivalent volumes of rabbit antisera [anti-FusC] (diluted 1/500 in PBST) for 1 hr, prior to addition to microplates. (Note: on occasion TAFC or FusC calibrator range was 0-20 µg/ml). Following addition, and incubation on microplates at room temperature (18-22 °C) for 1 hr, reagents were removed by Phosphate Buffered Saline-0.05%(v/v) Tween-20® (PBST) washing (4x), followed by addition of IgG[anti-rabbit IgG]-horseradish peroxidase conjugate (1/1000 dilution; 100 µl/well) for 1 hr. After conjugate removal and PBST washing (4x), Tetramethylbenzidine (TMB) substrate (100 µl/well) was added for 15 min and the reaction terminated by addition of 1N H₂SO₄ (100 µl/well). Finally the absorbance was measured at 450/620 nm using a Synergy platereader and standard curves of A_{450/620nm} versus log FusC concentration (0-100 µg/ml (or 0-20 µg/ml TAFC) constructed, from which the concentration of FusC in normal clinical specimens, of animal and human origin, was computed.

Specimens were also assayed at multiple dilutions to assess assay parallelism. Negative sera were spiked with purified FusC and subjected to FusC ELISA analysis to confirm assay specificity.

### RESULTS

### 1. FusC & TAFC purification and characterisation

FusC (Figure 1) was purified from culture supernatants of *A. fumigatus* ATCC46645 by C₁₈ RP-HPLC (yield: 16.5 mg/100 ml culture supernatant. TAFC (Figure 1) was obtained commercially (cTAFC) and also purified from *A. fumigatus* ATCC46645 (ihTAFC) by XAD-2 column chromatography. The structures of the non-ferrated and ferrated forms of FusC and TAFC are shown in Figure 1. Purity of resultant FusC was assessed by RP-HPLC and it can be seen from Figure 2 that it elutes with a Retention time (Rt) = 10.976 min. MALDI-ToF mass spectrometry was used to unambiguously confirm the identity of FusC and it can be seen from Figure 2 and Table 2 that the expected mass:charge ratio (m/z), equivalent to molecular mass, was obtained. RP-HPLC analysis to ihTAFC, which confirmed TAFC and FusC presence in this preparation, is also shown (Figure 2).

**Table 2: Fusarinine C (FusC) m/z data from MALDI-ToF analysis.**

| **Siderophore** | **m/z** | |
|---|---|---|
| | **Predicted** | **Observed** |
| Fusarinine C | 726 | 726.464 |
| Fusarinine C (Na⁺ adduct) | 748 | 748.513 |
| Fusarinine C + Fe³⁺ | 779.636 | 779.457 |
| Fusarinine C (Na⁺ adduct) + Fe³⁺ | 802 | 801.447 |

### 2. Protein-siderophore conjugate analysis

SDS-PAGE analysis indicated the presence of TAFC/FusC-sHSAB-BSA conjugates (*PSC1C&D*) as increased molecular mass species were only evident when siderophore was present in the conjugation mixture (Figure 2). Moreover, CAS analysis of *PSC1A, B* (data not shown) & *D* (Figure 2) confirmed the presence of bound siderophore whereby iron removal from the ferrated CAS reagent was enhanced only when *PSC1* was present. Neither BSA or KLH, or sHSAB-activated protein, facilitated iron removal from CAS reagent and so produced no colour change in the CAS assay. To our knowledge, CAS reagent has not previously been used to confirm siderophore-protein conjugation.

Immunological analysis of immobilised protein conjugates (sHSAB-BSA or TAFC-sHSAB-BSA (*PSC1C*); 2 - 0.08 µg (80 ng)), using murine antisera (anti-KLH-sHSAB-TAFC (*PSC1A*&*B*)) was undertaken (Figure 2). No reactivity towards sHSAB-BSA is evident, while TAFC is recognised when bound to protein. Moderate inhibition of anti-TAFC antibody binding is evident, especially at 0.08 µg TAFC-sHSAB-BSA *(PSC1C),* in the presence of purified TAFC (ihTAFC) (Figure 2). FusC contains three NH₂ groups and activation was performed using a reduced molar excess of SMCC (SMCC: NH₂ groups, 0.16) in order to minimise reaction with all three amino groups (Figure 1), and consequent conjugate polymerisation.

Analysis of three *PSC2* & 3 conjugates is presented:
Conjugate A = Acetylated *PSC3A&B* (Activated & acetylated FusC:SATA-cBSA, 13.5:5.66 mg);
Conjugate B = *PSC2A&B* (Activated FusC:SATA-cBSA, 13.5:5.66 mg);
Conjugate C = *PSC2A&B* (Activated FusC:SATA-cBSA, 0.5:0.21 mg).

SDS-PAGE confirmed the formation of *PSC2A* conjugates (i.e., SMCC-activated FusC coupled to SATA-cBSA) whereby the appearance of high Mr conjugate formation (120 kDa) was only evident when SMCC-activated FusC was incorporated into the reaction mixture (Figure 3, lanes 1-4, 6). This conjugate was absent when SATA-cBSA or SMCC-SATA-cBSA were subjected to SDS-PAGE analysis (Figure 3, lanes 5, 7 and 8). CAS analysis of *PSC2A* conjugates confirmed FusC presence (Figure 3). Here, the estimated amount of conjugated FusC, expressed in terms of desferrioxamine (DFO) equivalents was: Conjugate A: 753 µg/ml (DFO equivalents); Conjugate A2: 280 µg/ml; Conjugate B: 336 µg/ml; Conjugate B2: 570 µg/ml; Conjugate C: 140 µg/ml. It appears that acetylation enhanced either activated FusC conjugation to SATA-cBSA, or Fe³⁺ affinity, since Conjugate A exhibits a higher DFO equivalent concentration than Conjugate B. In fact, the FusC:BSA was calculated to be 100:1 and 63, respectively, for Conjugates A and B (Table 3). No colour change upon SideroTec Assay™ analysis (i.e., FusC presence) was detectable for BSA, SATA-cBSA or SMCC-SATA-cBSA (Figure 3, Table 3).

**Table 3: Protein-siderophore conjugate (PSC) analysis using Chrome Azurol S assay.**

| **Sample** | **Mean OD 630 nm** | **[Siderophore] (µg/ml)** | **[cBSA] (µg/ml)** | **Siderophore/cBSA*** |
|---|---|---|---|---|
| cBSA | 0.8075 | 0 | 500 | 0 |
| Conjugate A | 0.206 | 753 | 480 | 100:1 |
| Conjugate B | 0.381 | 336 | 550 | 63:1 |
| Conjugate B2 | 0.2465 | 570 | 550 | 100:1 |
| Conjugate C | 0.582 | 140 | 170 | 77:1 |
| Conjugate A2 | 0.4295 | 280 | 167 | 154:1 |
| SMCC | 0.826 | 0 | 500 | 0 |
| SATA-cBSA | 0.821 | 0 | 500 | 0 |

| | | | | |
|---|---|---|---|---|
| * Based on Mᵣ FusC & BSA equivalent to 726 and 66000 Da, respectively. | | | | |

Direct assessment of Fe³⁺-binding to *PSC2A* conjugates also confirmed FusC presence, as the red-brown colour change occurs only as a result of Fe³⁺ chelation by FusC (Figure 4). Antisera produced against *PSC1A* conjugates (KLH-sHSAB-ihTAFC), was observed to specifically react with *PSC2* conjugates by immunoblot analysis (Figure 4). Since different carrier protein and linker reagents (KLH and sHSAB, respectively) were used for *PSG1A*&*B* preparation, the observed reactivity is unambiguously confirmed to be directed towards the FusC moiety. Overall, these data definitively confirm that FusC was covalently coupled to BSA and that Fe³⁺⁻binding affinity is intact for *PSC2* & *PSC3* conjugates.

### 3. FusC/TAFC ELISA development

When the antigen immobilised on the solid phase was prepared using identical cross-linker chemistry to that used for immunogen synthesis, extensive experimentation indicated that it was not readily possible to detect FusC, or TAFC, by competitive ELISA (Table 4). For example, although KLH-sHSAB-TAFC/FusC was found to be a successful immunogen, it was determined that the same cross linker chemistry did not produce a useful antigen for immobilisation. Therefore, it has now advantageously been found that certain specific antigens are suitable for detecting FusC or TAFC by competitive ELISA.

**Table 4: Detection of FusC or TAFC by competitive ELISA. Use of different cross-linker chemistry, and carrier protein, was necessary to enable detection of FusC using rabbit antisera, or TAFC using rabbit and murine antisera, by ELISA.**

| **Immunogen** | **ELISA antigen** | **FusC/TAFC Detection** |
|---|---|---|
| KLH-sHSAB-TAFC/FusC | BSA-sHSAB-TAFC/FusC(ihTAFC) | No |
| cBSA-SATA-SMCC-FusC | cBSA-SATA-SMCC-FusC | No |
| KLH-sHSAB-TAFC/FusC | cBSA-SATA-SMCC-FusC | Yes |

For example, it was observed that FusC or TAFC, respectively, could be detected by ELISA using murine antisera [anti-KLH-sHSAB-ihTAFC or cTAFC] (*PSC1A* & *PSC1B*) (dilution 1/1000) and immobilised BSA-SATA-SMCC-FusC (± acetylation; + Fe³⁺) (*PSC2B and PSC3B*) (Figure 5). The dynamic range of this ELISA (ELISA Format 1; EF1) was 0.05-5 µg/ml TAFC or FusC, respectively. Moreover, competitive ELISA formats were developed using this antisera whereby the immobilised antigen was either FusC (referred to hereinafter as ELISA format 2 (EF2)) or *PSC2B* and *PSC3B* (ELISA Format 3 (EF3)) (Figures 6, 7 & 8). Rabbit antisera [anti-KLH-sHSAB-ihTAFC; *PSC1A*] which could specifically detect conjugated FusC (as part of *PSC2*) was also identified. Free FusC 6.5 µg/ml prevents antibody (anti-*PSC1A*, rabbit antisera) recognition of conjugate *PSC2A* (cBSA-SATA-SMCC-FusC). This confirms that acetylated FusC, as part of *PSC3A* or *PSC3B,* is not essential for FusC detection. Thus, although EF3 uses a combination of *PSC2B* and *PSC3B* on the solid phase, *PSC2A* or *B* alone is sufficient for FusC detection (Figure 5B). The dynamic range of EF2 and 3, respectively, was found to be 0 - 100 µg/ml (Figure 6). The IC₅₀ for FusC detection was calculated by determination of the FusC concentration yielding 50% decrease in maximum absorbance/binding of IgG[anti-FusC] to immobilised FusC (Figure 6).

### 4. FusC ELISA validation and clinical specimen analysis

Availability of the FusC-specific ELISAs (formats EF2 and EF3, respectively) facilitated evaluation of analyte presence in normal and disease-state specimens of animal origin. Using either assay format, FusC was detectable in the urine and sera of immunocompromised guinea pigs experimentally inoculated with *A. fumigatus* (*n* = 5) (Figure 7 & 8). No detectable FusC levels were present in control animals (uninfected guinea pigs or infected, but immunocompetent guinea pigs) (Figure 7 and 8; i.e., Day 0 specimens when animals are either not exposed to *Aspergillus fumigatus,* or infection has not commenced. Figure 8 shows the time-course of FusC appearance and detection in the urine of immunocompromised and infected guinea pig urine. This is the first demonstration of FusC, or any siderophore as a biomarker of *A. fumigatus,* or any fungal disease in animals.

Significantly elevated FusC levels (mean ± S.D.= 193.5 ± 97 µg/ml; range: 83.5 - 267 µg/ml) were also detectable in human sera obtained from IA patients (*n* = 3) which confirms that FusC is present and detectable in clinical specimens of human origin using EF2 or EF3 assay formats. Sera obtained from uninfected individuals did not contain significant levels of FusC (mean ± S.D. = 10.9 ± 3.2 µg/ml; range: 3.27 - 13.74 µg/ml). Indeed the detection of low levels of FusC in normal human sera may be due to endogenous low levels of FusC or TAFC due to environmental exposure to *A. fumigatus* or minor cross-reactivity with endogenous human-specific analytes, or indicates a requirement for minor readjustment of ELISA cutoff for negative:positive discrimination. FusC was detectable using EF2 following prior adulteration of normal sera with purified FusC, moreover, the recovery of FusC was proportional to specimen dilution which serves to validate EF2 performance with respect to FusC detection (Table 5). Dilution of clinical sera, likewise, yielded a proportional increase in observed A_{450/620 nm} which corresponds to decreased FusC concentrations (Table 5).

**Table 5: Assay validation data for detection of FusC in human sera. Specimen dilution (1/10) results in optimal FusC recovery (97-100 % in spiked normal (J18) and clinical specimens (Clin 1-3 specimens from IA patients), respectively). Mean % recovery, or linearity of dilution, for FusC in clinical specimens = 117% (1/10-1/40 dilution) which indicates optimal assay performance.**

| **Specimen ID & dilution** | **[FusC] µg/ml** | | |
|---|---|---|---|
| | **Uncorrected (in microwell)** | **Corrected (x Dilution Factor)** | **% Recovery** |
| **J18 (Spiked Normal Serum)** | | | |
| 1/10 | 77.8 | 778 | 97 |
| 1/20 | 45 | 900 | 112 |
| 1/40 | 28 | 1120 | 140 |
| 1/80 | 16 | 1280 | 160 |

| **IPA specimens:** | | | |
|---|---|---|---|
| **Clin 1** | | | |
| 1/10 | 7.15 | 71.5 | 100 |
| 1/20 | 4.35 | 87 | 120 |
| 1/40 | 3 | 120 | 167 |

| **Clin 2** | | | |
|---|---|---|---|
| 1/10 | 16.87 | 168.7 | 100 |
| 1/20 | 7.46 | 149.2 | 88 |
| 1/40 | 5.18 | 207 | 122 |

| **Clin 3** | | | |
|---|---|---|---|
| 1/10 | 19.7 | 197 | 100 |
| 1/20 | 9.6 | 192 | 97 |
| 1/40 | 5.6 | 224 | 113 |

Advantageously, this is the first demonstration of (i) antisera generation against either TAFC or FusC, (ii) the detection of either analyte by ELISA, more specifically by competitive ELISA, and (iii) the detection of a fungal siderophore (i.e., FusC) in disease state specimens (e.g., serum, plasma or urine) of animal (i.e., human or guinea pig) origin. Accordingly, the present invention advantageously provides a method for detecting infections caused by or associated with siderophore-secreting microorganisms, including, but not limited to, infections caused by or associated with *Aspergillus fumigatus,* especially aspergillosis and more specifically invasive pulmonary aspergillosis (IPA), which is simple, accurate and cost-efficient.

### Example 2

### Preparation of FusC-BSA-AuNP conjugates as labels and solid phases for competitive immunoassays

### Method

### 1. Preparation of OD 2.0 Colloidal Gold

6 ml AuNP (O.D ≈ 0.7) was added to 12 x Sigma-coated Eppendorf tubes (500 µl each). The Eppendorfs were then centrifuged at 14,000 x g for 25 min, 4°C. Supernatant was removed and the pellets were pooled then together and resuspended in 2 mM borate (pH 9.0) to yield an optical density of 2.0 at a wavelength of 520 nm.

### 2. Conjugation of FusC-BSA to Gold Nanoparticles (AuNP)

In order for successful conjugation, the appropriate conditions for the AuNP and FusC-BSA attraction had to be determined. Following stability testing, pH 9.0 was determined to be the optimum pH; and 350 µg/ml FusC-BSA was determined to be the optimum concentration for conjugate formation. 0.5 ml AuNP (O.D 2.0) in 2 mM borate (pH 9.0) was added to 0.5 ml (175 µg) FusC-BSA (also in 2 mM borate) in a Sigma-coated Eppendorf tube and allowed to incubate for 20 min at room temperature. A 50 µl aliquot of the AuNP-FusC-BSA conjugate was deemed stable (remained red in colour) when the solution was brought to 0.1 M NaCl using 2 M NaCl. The conjugate was then passivated and stabilised with 250 µl 10 mg/ml BSA in 2 mM borate and allowed to incubate at room temperature for 60 min. The samples were then centrifuged at 14,000 x g for 15 min at 4 °C. The supernatant was discarded and the nanoparticle residue was redispersed in 500 µl 2 mM borate. This step was repeated in order to clear unbound FusC-BSA from the sample. The samples were centrifuged again (14,000 x g for 15 min, 4 °C) and redispersed in 250 µl 0.01 M phosphate buffer with 0.1 M NaCl (0.01 M Na₂HPO₄ brought to pH 7.4 using 0.01 M NaH₂PO₄). The final, stable conjugate was then stored in Sigma-coated Eppendorf tubes at 4 °C. A negative control Au-BSA conjugate was prepared by adding 0.5 ml (175 µg) BSA to 0.5 ml AuNP, and following the procedure identical to the one used for the Au-FsC-BSA conjugate preparation.

### 3. Confirmation of Au-FsC-BSA Conjugate Formation

Confirmation of conjugate formation was performed using a protein immunoblot. 4 µl of AuNP-FsC-BSA, Au-BSA and FusC-BSA samples were spotted onto a nitrocellulose membrane strip in serial dilutions (A-E) prepared in in 2 mM borate (pH 9.0). Spots were allowed to dry before the strip was blocked for 30 min in Marvel (5 % (w/v) in PBS). Membranes were then incubated for 30 min with primary antibody (rabbit Anti-FusC), diluted using blocking buffer (1/1000). Nitrocellulose blots were subsequently washed (2 x 5 min) in blocking solution and then incubated with a 1/1000 dilution of anti-rabbit IgG peroxidase-conjugated secondary antibody (Sigma-Aldrich), shaking for 30 min at room temperature. The nitrocellulose membranes were then washed extensively (2 x 5 min) in blocking solution and rinsed (2 x 5 min) with PBS. Immunoreactive samples were visualized using Pierce ECL Chemiluminescent Substrate (Fischer Scientific) for the detection of horseradish peroxidase.

### Results

Figure 9 shows a protein immunoblot of FusC-BSA-AuNP (A), BSA-AuNP (B) and FusC-BSA (C). High reactivity against FusC is clearly evident for the FusC-BSA conjugate across all concentrations. Reactivity is also visible for the FusC-BSA-AuNP conjugate - confirming the conjugation of FusC-BSA to gold nanoparticles. No reactivity is visible for the BSA-AuNP control.

The immunoblot result confirmed that gold nanoparticles were successfully conjugated to FusC-BSA. Conjugation of FusC-BSA to the AuNP was most likely achieved through electrostatic adsorption. As the bright red colour of the conjugate and Au-BSA control interfered with the signal detection, the chemiluminescent substrate ECL was used to detect the secondary antibody instead of a chromogenic substrate.

## Claims

1. A method for detecting fusarinine C (FusC), 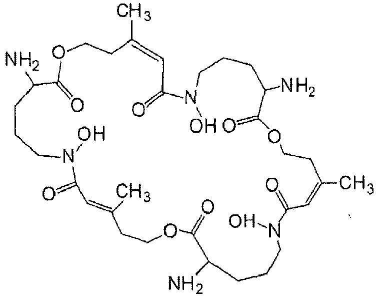 and/or detecting infections caused by or associated with FusC- secreting microorganisms, preferably an *Aspergillus fumigatus* infection, more preferably invasive aspergillosis (IA), in a biological sample of a subject, the method comprising:
providing a solid support having either bound FusC or a conjugate thereof, or bound anti-FusC antibody;
reacting the bound FusC or a conjugate thereof, with an anti-FusC antibody, and a biological sample of a subject; or reacting the bound anti-FusC antibody with a FusC or conjugate thereof, and a biological sample of a subject;
detecting and/or quantifying the presence of FusC in the biological sample,
wherein the presence of FusC in the biological sample is indicative of infection caused by or associated with FusC-secreting microorganisms, preferably *Aspergillus fumigatus* infection, more preferably invasive aspergillosis (IA) and wherein the anti-FusC antibody was raised against an immunogen comprising FusC covalently coupled to a carrier protein.

2. The method of claim 1 wherein the carrier protein is keyhole limpet haemocyanin (KLH) or bovine serum albumin (BSA), preferably cationised BSA (cBSA).

3. The method according to claim 2 wherein the immunogen is selected from KLH-sHSAB-FusC, cBSA-SATA-SMCC-FusC and cBSA-SATA-SMCC-FusC(Fe³⁺).

4. A kit for use in detecting FusC, 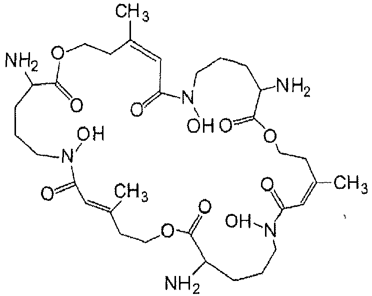 and/or detecting infections caused by or associated with FusC-secreting microorganisms, preferably an *Aspergillus fumigatus* infection, more preferably invasive aspergillosis (IA), in a biological sample of a subject comprising:
a. a solid support having bound FusC or a conjugate thereof; and
b. an anti-FusC antibody wherein the anti-FusC antibody was raised against an immunogen comprising FusC covalently coupled to a carrier protein.

5. A kit for use in detecting FusC, and/or detecting infections caused by or associated with FusC-secreting microorganisms, preferably an *Aspergillus fumigatus* infection, more preferably invasive aspergillosis (IA), in a biological sample of a subject comprising:
a. a solid support having bound an anti-FusC antibody wherein the anti-FusC antibody was raised against an immunogen comprising FusC covalently coupled to a carrier protein; and
b. FusC or a conjugate thereof.

6. The kit of claim 4 or 5 comprising at least one antibody as defined in any one of claims 1 to 3.

## Patentansprüche

1. Verfahren zum Nachweis von Fusarinin C (FusC), 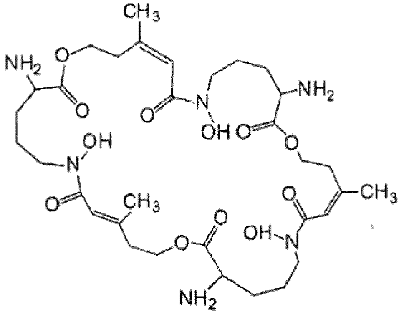 und/oder Nachweis von Infektionen, die durch FusC-sezernierende Mikroorganismen verursacht werden oder mit diesen assoziiert sind, vorzugsweise einer *Aspergillus fumigatus*-Infektion, stärker bevorzugt von invasiver Aspergillose (IA) in einer biologischen Probe eines Individuums, wobei das Verfahren umfasst:
Bereitstellen eines festen Trägers mit entweder gebundenem FusC oder einem Konjugat davon oder gebundenem Anti-FusC-Antikörper;
Umsetzen des gebundenen FusC oder eines Konjugats davon mit einem Anti-FusC-Antikörper und einer biologischen Probe eines Individuums; oder Umsetzen des gebundenen Anti-FusC-Antikörpers mit einem FusC oder Konjugat davon und einer biologischen Probe eines Individuums;
Nachweisen und/oder Quantifizieren der Anwesenheit von FusC in der biologischen Probe, wobei die Anwesenheit von FusC in der biologischen Probe auf eine Infektion hindeutet, die durch FusC-sezernierende Mikroorganismen verursacht wird oder damit assoziiert ist, vorzugsweise *Aspergillus fumigatus*-Infektion, stärker bevorzugt invasive Aspergillose (IA), und wobei der Anti-FusC-Antikörper gegen ein Immunogen erzeugt wurde, das kovalent an ein Trägerprotein gebundenes FusC umfasst.

2. Verfahren nach Anspruch 1, wobei das Trägerprotein Schlüssellochnapfschnecken-Hämocyanin (KLH) oder Rinderserumalbumin (BSA), vorzugsweise kationisiertes BSA (cBSA) ist.

3. Verfahren nach Anspruch 2, wobei das Immunogen ausgewählt ist aus KLH-sHSAB-FusC, cBSA-SATA-SMCC-FusC und cBSA-SATA-SMCC-FusC(Fe³⁺).

4. Kit zur Verwendung beim Nachweis von FusC, 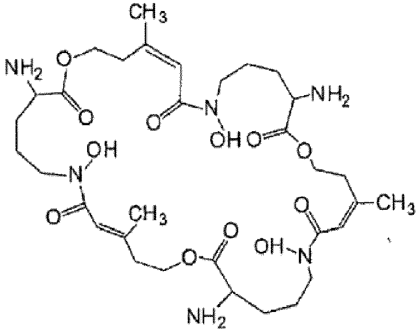 und/oder Nachweis von Infektionen, die durch FusC-sezernierende Mikroorganismen verursacht werden oder mit diesen assoziiert sind, vorzugsweise einer *Aspergillus fumigatus*-Infektion, stärker bevorzugt invasiver Aspergillose (IA) in einer biologischen Probe eines Individuums, umfassend:
a. einen festen Träger mit gebundenem FusC oder einem Konjugat davon; und
b. einen Anti-FusC-Antikörper, wobei der Anti-FusC-Antikörper gegen ein Immunogen erzeugt wurde, das kovalent an ein Trägerprotein gebundenes FusC umfasst.

5. Kit zur Verwendung beim Nachweis von FusC und/oder zum Nachweis von Infektionen, die durch FusC-sezernierende Mikroorganismen verursacht werden oder damit assoziiert sind, vorzugsweise einer *Aspergillus fumigatus*-Infektion, vorzugsweise invasiver Aspergillose (IA), in einer biologischen Probe eines Individuums, umfassend:
a. einen festen Träger mit einem gebundenen Anti-FusC-Antikörper, wobei der Anti-FusC-Antikörper gegen ein Immunogen erzeugt wurde, das kovalent an ein Trägerprotein gebundenes FusC umfasst; und
b. FusC oder ein Konjugat davon.

6. Kit nach Anspruch 4 oder 5, umfassend mindestens einen Antikörper nach einem der Ansprüche 1 bis 3.

## Revendications

1. Procédé de détection de fusarinine C (FusC), 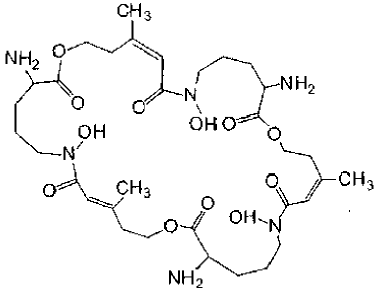 et/ou de détection d'infections causées par ou associées à des micro-organismes sécrétant FusC, de préférence une infection par *Aspergillus fumigatus,* plus préférablement une aspergillose invasive (AI), dans un échantillon biologique d'un sujet, le procédé comprenant :
la fourniture d'un support solide auquel est lié FusC ou un conjugué de celui-ci, ou un anticorps anti-FusC ;
la réaction du FusC ou un conjugué de celui-ci lié, avec un anticorps anti-FusC, et un échantillon biologique d'un sujet ; ou la réaction de l'anticorps anti-FusC lié avec un FusC ou un conjugué de celui-ci, et un échantillon biologique d'un sujet ;
la détection et/ou la quantification de la présence de FusC dans l'échantillon biologique,
dans lequel la présence de FusC dans l'échantillon biologique est indicative d'une infection causée par ou associée à des microorganismes sécrétant FusC, de préférence une infection par *Aspergillus fumigatus,* plus préférablement une aspergillose invasive (AI) et dans lequel l'anticorps anti-FusC a été produit contre un immunogène comprenant FusC couplé de façon covalente à une protéine de support.

2. Procédé selon la revendication 1, dans lequel la protéine de support est l'hémocyanine de patelle (KLH) ou la sérum-albumine bovine (BSA), de préférence BSA cationisée (cBSA).

3. Procédé selon la revendication 2, dans lequel l'immunogène est choisi parmi KLH-sHSAB-FusC, cBSA-SATA-SMCC-FusC et cBSA-SATA-SMCC-FusC(Fe³⁺).

4. Trousse pour utilisation pour détecter FusC, 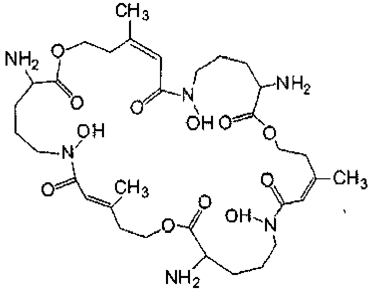 et/ou détecter des infections causées par ou associées à des micro-organismes sécrétant FusC, de préférence une infection par *Aspergillus fumigatus,* plus préférablement une aspergillose invasive (AI), dans un échantillon biologique d'un sujet comprenant :
a. un support solide auquel est lié FusC ou un conjugué de celui-ci ; et
b. un anticorps anti-FusC, l'anticorps anti-FusC ayant été produit contre un immunogène comprenant FusC couplé de façon covalente à une protéine de support.

5. Trousse pour utilisation pour détecter FusC, et/ou détecter des infections causées par ou associées à des micro-organismes sécrétant FusC, de préférence une infection par *Aspergillus fumigatus,* plus préférablement une aspergillose invasive (AI), dans un échantillon biologique d'un sujet comprenant :
a. un support solide auquel est lié un anticorps anti-FusC, l'anticorps anti-FusC ayant été produit contre un immunogène comprenant FusC couplé de façon covalente à une protéine de support ; et
b. FusC ou un conjugué de celui-ci.

6. Trousse selon la revendication 4 ou 5 comprenant au moins un anticorps tel que défini dans l'une quelconque des revendications 1 à 3.
